**Europäisches Patentamt**

(19) **European Patent Office**

**Office européen des brevets**

(11) Veröffentlichungsnummer: **0 017 608**
**B1**

(12) # EUROPÄISCHE PATENTSCHRIFT

(45) Veröffentlichungstag der Patentschrift:
**04.05.83**

(51) Int. Cl.³: **A 61 N 1/36,** A 61 N 1/08,
G 01 R 31/36

(21) Anmeldenummer: **80730021.5**

(22) Anmeldetag: **06.03.80**

(54) **Schaltung zur Kontrolle des Batteriezustands bei einem Herzschrittmacher.**

(30) Priorität: **31.03.79 DE 2913399**

(43) Veröffentlichungstag der Anmeldung:
**15.10.80 Patentblatt 80/21**

(45) Bekanntmachung des Hinweises auf die Patenterteilung:
**04.05.83 Patentblatt 83/18**

(84) Benannte Vertragsstaaten:
**AT CH DE FR GB IT NL SE**

(56) Entgegenhaltungen:
**EP-A-0 011 949**
**DE-A-2 461 342**
**DE-A-2 518 038**
**FR-A-2 117 641**

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **Blaser, Reinhard, Dr,-Ing., Fasanenweg 15, D-6470 Büdingen 2 (DE)**

(74) Vertreter: **Christiansen, Henning, Dipl.-Ing., Unter den Eichen 108a, D-1000 Berlin 45 (DE)**

## Schaltung zur Kontrolle des Batteriezustands bei einem Herzschrittmacher

Die Erfindung betrifft eine Schaltung zur Kontrolle des Batteriezustands bei einem Herzschrittmacher mit einem Spannungsdetektor, der ein Signal abgibt, sobald eine den Batteriezustand charakterisierende Größe einen Grenzwert passiert.

Derartige Schaltungen dienen bei künstlichen Herzschrittmachern zur Anzeige des herannahenden Endes der Betriebszeit (EOL) der Batterie, so daß rechtzeitig die notwendigen Maßnahmen getroffen werden können. Eine verbreitete Möglichkeit zur Kontrolle des Batteriezustands besteht darin, daß der Generator des Herzschrittmachers zur Erzeugung von Stimulationsimpulsen eine mit abnehmender Batteriespannung fallende Frequenzkennlinie bei Betrieb mit fester Impulsrate aufweist. Unterschreitet die Impulsrate einen vorgegebenen Wert, so ist ein Austausch erforderlich.

Bei der Verwendung von CMOS-Schaltungen oder quarzgesteuerten Oszillatoren ändert sich die Frequenz nicht nennenswert mit der Batteriespannung. Um trotzdem eine Aussage über den Batteriezustand zu ermöglichen, ist es bekannt, in Schaltungen von Herzschrittmachern zusätzliche spannungsabhängige Triggerschaltungen vorzusehen, die ein Signal abgeben, wenn die Batteriespannung einen vorgegebenen Wert unterschreitet. Bei diesen bekannten Schaltungen wird lediglich die Klemmenspannung der Batterie bewertet. Die Klemmenspannung ist aber wegen des Innenwiderstandes der Batterie vom durch den Betriebszustand des Schrittmachers bedingten Stromverbrauch abhängig. Da insbesondere programmierbare Schrittmacher in ihrem Betriebsverhalten in weiten Bereichen veränderlich sind, schwankt der zugehörige Stromverbrauch entsprechend. Schon bei herkömmlichen Demand-Schrittmachern ändert sich der Stromverbrauch beim Übergang vom inhibierten auf den stimulierenden Betrieb im Verhältnis 5 : 1.

In einem Grenzbereich gegen Ende der Betriebszeit der Batterie kann damit der Zustand eintreten, daß in einer Betriebsart bereits das herannahende Ende der Batterielebensdauer angezeigt wird, während das in einer anderen Betriebsart noch nicht der Fall ist. Hiermit ist die Gefahr verbunden, daß, wenn der Schrittmacher sich längere Zeit ununterbrochen in einem relativ inaktivem Betriebszustand befindet noch nichts auf die bevorstehende Erschöpfung der Batterie hinweist, so daß, wenn — bedingt durch das Herzverhalten — eine längere Betriebsphase mit größerer Schrittmacheraktivität erforderlich ist, zwar ein entsprechendes Warnsignal ausgegeben wird, das Ende der Betriebszeit der Batterie aber bedrohlich schnell erreicht wird, da sie sich im vorhergehenden Betriebszustand unbemerkt bereits zu sehr erschöpft hatte.

Wenn das von einer derartigen Schaltung zur Anzeige des Batteriezustands abgegebene Signal dazu benutzt wird, um die Grundrate des — an sich betriebsspannungsunabhängigen — Oszillators durch interne Umschaltungen herabzusetzen, so wird der mittlere Stromverbrauch des Schrittmachers vermindert und die durch Siebglieder geglättete Batterie-Klemmenspannung steigt erneut an. Damit wird durch die Kontrollschaltung aber kein Signal mehr abgegeben und die Grundrate des Schrittmachers wieder heraufgesetzt. Durch diese Rückkopplungswirkung wird auf diese Weise ein fortwährender Frequenzwechsel hervorgerufen, der für den Schrittmacherträger außerordentlich störend ist.

Der im Anspruch 1 angegebenen Erfindung liegt die Aufgabe zugrunde, eine Schaltung der vorgenannten Gattung anzugeben, die eine Aussage über den Batteriezustand unabhängig vom derzeitigen Betrieb des Schrittmachers ermöglicht. Die Schaltung soll weiterhin an unterschiedliche Batterietypen derart anpaßbar sein, daß in jedem Fall die auf das Ende der Betriebszeit des jeweiligen Batterietyps hindeutenden Anzeichen sicher erfaßt und ausgewertet werden.

Besonders günstig ist bei der erfindungsgemäßen Schaltung, daß nicht nur der Abfall der Klemmenspannung der Batterie als Zeichen ihrer Erschöpfung erkannt wird, sondern auch ihr Innenwiderstand als Kriterium herangezogen wird, wobei betriebsbedingte Änderungen des Stromverbrauchs der Schrittmacherschaltung keine Rolle spielen. Auf diese Weise ist es möglich, auch eine — selten vorkommende — unzulässige Erhöhung des Innenwiderstandes der Batterie sofort zu erkennen und daraufhin ein entsprechendes, Aufmerksamkeit hervorrufendes Signal auszulösen, ohne daß erst ein Betriebszustand eintreten muß, der durch entsprechend großen Stromverbrauch auch die Klemmenspannung unter den Schwellwert absinken läßt. Es ist nämlich hier durchaus der Fall denkbar, daß der Innenwiderstand der Batterie sich bereits soweit erhöht hat, daß ein derartiger Betriebszustand mit größerem Stromverbrauch überhaupt nicht mehr aufrechterhalten werden kann.

Bei der erfindungsgemäßen Schaltung erfolgt die Steuerung der Mittel zum Trennen und Verbinden der Batterie bevorzugterweise durch ein einziges Signal, wobei zu definierten Zeitpunkten ein Umschalten der Batterie vorgenommen wird.

Vorteilhafte Weiterbildungen der Erfindung sind in den Unteransprüchen angegeben und werden nachfolgend anhand eines Ausführungsbeispiels zusammen mit den übrigen Merkmalen der Erfindung näher erläutert.

Es zeigt

Fig. 1 einen Stromlaufplan der erfindungsgemäßen Schaltung und

Fig. 2 eine Darstellung des zeitlichen Verlaufs verschiedener Spannungen in der Schaltung gemäß Fig. 1.

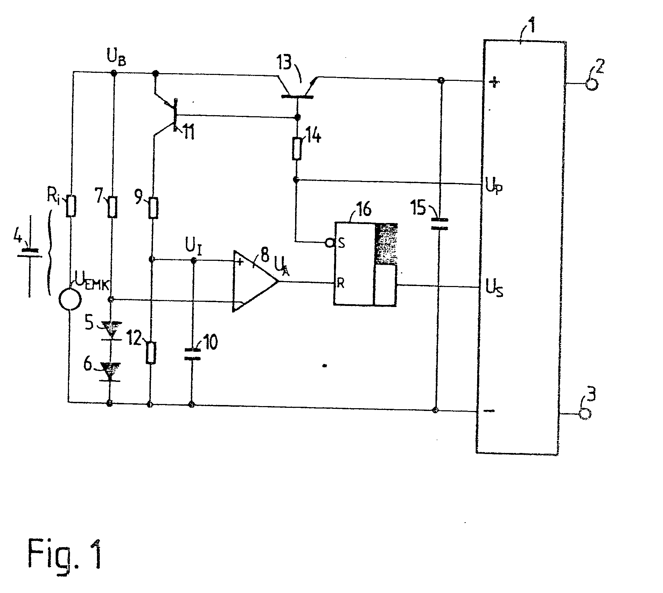

In dem in Fig. 1 wiedergegebenen Stromlaufplan ist die eigentliche Schaltung des Herzschrittmachers zur Erzeugung von Stimulationsimpulsen insgesamt als Block 1 dargestellt. Anschlüsse 2 und 3 dienen dabei als Verbindungspunkte für die Signalübertragung zum und vom Herzen. Der links von Block 1 detailliert wiedergegebene Teil der Schrittmacherschaltung enthält eine die Stromversorgung bildende Batterie 4, an deren Klemmen die Spannung $U_B$ anliegt und die in der Schaltung ein zweites Mal, zerlegt in die Spannungsquelle $U_{EMK}$ und ihren Innenwiderstand $R_i$, wiedergegeben ist. Die zwischen der Batterie 4 und dem Block 1 angeordneten Bauelemente bilden die erfindungsgemäße Schaltung zur Überwachung des Batteriezustands.

Zwei in Flußrichtung geschaltete Dioden 5 und 6, die über einen Widerstand 7 von der Batterie 4 her von Strom durchflossen werden, bilden eine Konstantspannungsquelle. Da die Betriebstemperatur des Schrittmachers durch die Körpertemperatur im wesentlichen konstant gehalten wird, sind bei dieser Schaltung Mittel zur Temperaturkompensation dann nicht erforderlich, wenn der Abgleich der Schaltung bzw. ihre Dimensionierung für 37° C erfolgt. Die Spannung an den Dioden 5 und 6 wird dem invertierenden Eingang eines Differenzverstärkers 8 zugeführt, an dessen nichtinvertierenden Eingang die durch ein aus einem Widerstand 9 und einem Kondensator 10 gebildetes RC-Glied integrierte Spannung $U_I$ gelangt. Das RC-Glied wird über einen Transistor 11 zu vorbestimmten Zeiten mit der Klemmenspannung $U_B$ der Batterie 4 verbunden. Wenn der Transistor 11 gesperrt ist, wird der Kondensator 10 über einen Widerstand 12, dessen Wert groß ist gegenüber dem Wert des Widerstands 9, entladen. Die Bemessung des RC-Glieds ergibt sich aus der weiter unten folgenden Beschreibung der Funktion der Schaltung.

Zusammen mit dem Transistor 11 wird ein weiterer Transistor 13 über einen Widerstand 14 angesteuert, der allerdings im Vergleich zu dem Transistor 11 eine entgegengesetzte Polarität aufweist, so daß die beiden Transistoren bei identischem Ansteuerungssignal entgegengesetztes Schaltverhalten zeigen. Anstelle der Transistoren 11 und 13 können auch andere Schaltelemente Verwendung finden, die wahlweise sperrend und leitend wirken. Über den Transistor 13 wird der die eigentliche Stimulationsschaltung des Schrittmachers enthaltende Block 1 und die Siebmittel für die Versorgungsspannung — im dargestellten Ausführungsbeispiel ein Kondensator 15 — von der Spannungsquelle, d. h. der Batterie 4, getrennt. Die Ansteuerung der Transistoren 11 und 13 erfolgt über eine pulsierende Ausgangsspannung $U_P$, wobei der die Transistoren 11 und 13 ansteuernde Impuls zweckmäßigerweise aus der Stimulationsschaltung (Block 1) abgeleitet wird. Wenn der Block 1 eine CMOS- oder eine andere digitale Schaltung aufweist, läßt sich ein geeigneter Impuls aus den ohnehin vorhandenen Impulserzeugerschaltungen ableiten. Der Prüfzeitraum T kann durch Stimulations- bzw. CLOCK-Impulse ausgelöst werden. Voraussetzung ist lediglich, daß die Impulse konstante Zeitdauern aufweisen und mit maximalen Zeitabständen aufeinanderfolgen, die eine ausreichend häufige Überprüfung der Batterie gewährleisten. Die pulsierende Spannung $U_P$ liegt außerdem noch an dem Setzeingang S eines RS-Flip-Flops 16 an, dessen Rücksetz-Eingang R mit dem Ausgang des Differenzverstärkers 8 verbunden ist, wobei die Ausgangsspannung mit $U_A$ bezeichnet ist. Die am Ausgang des RS-Flip-Flops erscheinende Signalspannung $U_S$ gelangt zu dem Block 1 zurück, an dessen entsprechendem Eingang bei gesetztem Flip-Flop 16 ein das herannahende Ende der Batterielebensdauer kennzeichnendes Signal ausgelöst wird. Um diesen Zustand anzuzeigen, kann beispielsweise — wie bei bekannten Schrittmacherschaltungen — die Grundrate des Schrittmachers herabgesetzt werden, so daß der Schrittmacherträger oder der behandelnde Arzt auf den Batteriezustand aufmerksam wird. Die Herabsetzung der Grundrate erfolgt bei einem digital aufgebauten Impulsgenerator, wenn der Impulsgenerator beispielsweise jeweils bei Erreichen eines vorgegebenen Zählerstands in einen Ausgangszustand zurückgesetzt wird, durch eine entsprechende Beeinflussung der logischen Vergleichssignale.

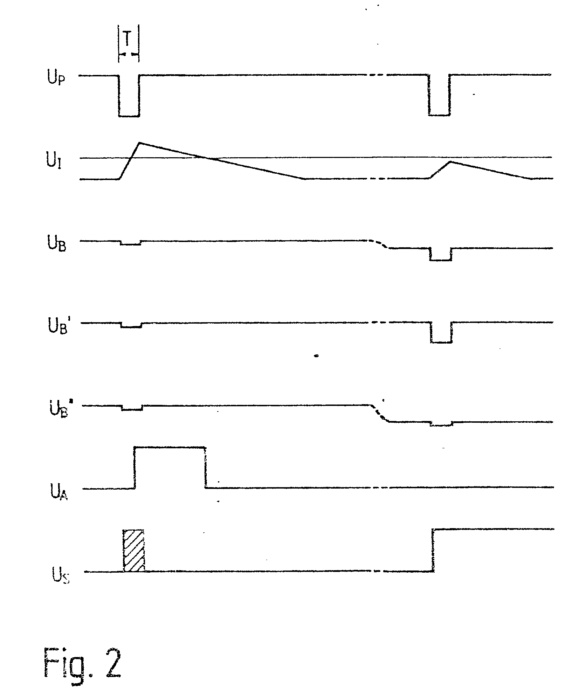

Die Funktion der Schaltung soll nunmehr anhand der in Fig. 2 wiedergegebenen Amplitudenverläufe der in Fig. 1 bezeichneten Signalspannungen beschrieben werden.

Durch die pulsierende Spannung $U_P$ werden die Prüfzeiträume für die Batterie festgelegt. Diese Zeiträume sind mit Stimulationsimpulsen korreliert oder werden durch andere Zeitgebermittel so gesteuert, daß sie ausreichend häufig auftreten. Durch die Spannung $U_P$ wird — wenn sie ihren positiven Maximalwert innehat — der Transistor 13 über seine Basis durch den Widerstand 14 leitend gehalten. (Der Wert der Spannung $U_P$ muß dazu über dem Wert der positiven Versorgungsspannung + des Blocks 1 liegen, was aber beispielsweise durch eine in Schrittmacherschaltungen übliche Spannungsvervielfacherschaltung erreicht werden kann.) Der Transistor 11 ist zunächst gesperrt. Erscheint im Verlauf der Schaltung $U_P$ ein negativer Impuls, so wird der Transistor 13 gesperrt und dafür der Transistor 11 durch den nunmehr über den Widerstand 14 zu seinem Basisanschluß gelangenden Strom leitend. Gleichzeitig wird das Flip-Flop 16 über seinen invertierenden Setz-Eingang S gesetzt. Über den Widerstand 9 wird der Kondensator 10 aufgeladen, wobei der über den Widerstand 12 abfließende Strom vernachlässigbar ist. Die Spannung $U_I$ am Kondensator 10 steigt im Verlauf des negativen Impulses an, wie es im Diagramm gemäß Fig. 2 sichtbar ist. Die RC-Kombination bildet dabei eine Last für die Batterie 4, die groß ist gegenüber dem Innenwiderstand $R_i$, wobei der Strom nicht aus dem Siebkondensator 15 geliefert werden kann, da dieser durch den gesperrten Transistor 13 von der Batterie getrennt ist. Auch bei neuer Batterie ist der Innenwiderstand $R_i$ größer als Null, so daß die Klemmenspannung $U_B$ einen Einbruch erleidet, wie es in Fig. 2 im linken Bereich der entsprechenden Diagramme $U_B$ bis $U_B''$ jeweils erkennbar ist. Bei ausreichend frischer Batterie übersteigt die Spannung $U_I$ am Kondensator 10 den Wert der an den Dioden 5 und 6 abfallenden Gesamtspannung (horizontale Linie im Diagramm für $U_I$ in Fig. 2), so daß am invertierenden Ausgang des Differenzverstärkers 8 eine ansteigende Impulsflanke entsteht (Spannung $U_A$), die das Flip-Flop 16 zurücksetzt. Die am Ausgang des Flip-Flops 16 erscheinende Signalspannung $U_S$ hat damit höchstens für die Zeitdauer des negativen Impulses der Spannung $U_P$ den logischen H-Pegel eingenommen, so daß kein bleibender, das bevorstehende Ende der Lebensdauer der Batterie anzeigender Signalzustand erscheint. Der undefinierte Bereich der Signalspannung $U_S$ ist in der Zeichnung schraffiert dargestellt.

Mit zunehmender Betriebsdauer der Batterie wird in der Regel die Leerlaufspannung $U_{EMK}$ absinken und der Innenwiderstand $R_i$ ansteigen. Das hat zur Folge, daß während des Prüfimpulses der Spannung $U_P$ die Spannung $U_I$ am Kondensator 10 nicht die an den Dioden 5 und 6 abfallende Schwellspannung überschreitet (rechter Teil der Spannungsverläufe gemäß Fig. 2). In diesem Fall wird das Flip-Flop 16 durch die abfallende Flanke der Spannung $U_P$ zwar gesetzt, da die Ausgangsspannung $U_A$ des Differenzverstärkers 8 sich während der Dauer des Prüfimpulses nicht ändert, wird das Flip-Flop 16 aber nicht mehr zurückgesetzt. Die Spannung $U_S$ behält ihren hohen Pegel bei und zeigt damit das herannahende Ende der Batterielebensdauer an, wobei durch dieses Signal geeignete Schaltungszustände innerhalb der übrigen Schrittmacherschaltung ausgelöst werden.

Das Ende der Lebensdauer eines Primärelementes kann einerseits dadurch bestimmt werden, daß die Betriebsspannung durch Verbrauch des für die Reaktion zur Verfügung stehenden Materials herabgesetzt wird. Andererseits wird die Betriebsfähigkeit der Batterie auch dadurch beschränkt, daß sich Reaktionsprodukte an den Elektroden niederschlagen und damit zur Erhöhung ihres Innenwiderstandes $R_i$ beitragen. Mit der Abnahme der Leerlaufspannung wird in der Regel — bedingt durch die chemischen Reaktionen — eine entsprechende Erhöhung des Innenwiderstandes $R_i$ einhergehen, so daß ein Spannungsverlauf der Klemmenspannung unter Prüfbedingungen, wie er in Fig. 2 unter $U_B$ im rechten Teil dargestellt ist, auftreten wird: einerseits ist die unbelastete Klemmenspannung herabgesetzt und andererseits ist der zusätzliche Einbruch unter Belastung gegenüber dem frischen Zustand vergrößert.

Nun kann es jedoch vorkommen, daß bei einer fehlerhaften Batterie der Innenwiderstand $R_i$ sich bereits vor dem üblicherweise zu erwartenden Ende der Betriebszeit unzulässig vergrößert. Diese Änderung des Innenwiderstandes ist mit einer normalen, unbelasteten Spannungsmessung an der Batterie zur Feststellung des Betriebszustandes nicht zu erfassen. Der in Fig. 2 bei $U_B'$ angegebene Verlauf zeigt jedoch, daß auch bei gleichgebliebener Leerlaufspannung der infolge angestiegenen Innenwiderstands $R_i$ vergrößerte Spannungseinbruch im rechten Teil des Diagramms ausreicht, um den Kondensator 10 während des Prüfimpulses nicht über die Schwellspannung der Dioden 5 und 6 hinaus aufzuladen.

Sollte sich andererseits lediglich die Leerlaufspannung $U_{EMK}$ während des Betriebs nennenswert verringert haben, ohne daß der Innenwiderstand $R_i$ entsprechend ansteigt, so wird dieser Zustand, der in Fig. 2 im rechten Teil des Spannungsverlaufs $U_B''$ angedeutet ist, ebenfalls sicher erkannt, da die Klemmenspannung $U_B''$ unter Belastung während des Prüfimpulses den Minimalwert erreicht hat, bei dem der Kondensator 10 nicht mehr ausreichend geladen wird.

Die Dimensionierung des RC-Gliedes 9, 10 muß damit unter Berücksichtigung des Widerstandes 12 und der Impulsdauer T in der Weise erfolgen, daß bei dem für den betreffenden Schrittmacher vorgesehenen Batterietyp einerseits allein bei Absinken der Klemmenspannung $U_{EMK}$ auf ihren minimal zulässigen Wert (bei zu einer frischen Batterie gehörigem Innenwiderstand $R_i$) und andererseits allein bei bis zu dem zulässigen Grenzwert vergrößerten Innenwiderstand $R_i$ (bei der ursprünglichen Leerlaufspannung $U_{EMK}$), das herannahende Ende der Betriebszeit der Batterie anzeigende Signal sicher ausgelöst wird.

Damit ist gewährleistet, daß sich auch bei auftretenden Zwischenwerten eine den tatsächlichen Verhältnissen entsprechende EOL-Anzeige für die Batterie ergibt, insbesondere, wenn sich bei der durch den Spannungsdetektor auszuwertende Spannung der zunehmende Innenwiderstand und die abnehmende Leerlaufspannung im wesentlichen linear überlagern. Der exponentielle Verlauf der Ladespannung des Kondensators 10 und der durch die Betriebsdaten der Batterie bedingten Änderungen sind dabei in ihrem Anfangsbereich — bei ausreichend kleiner Vergleichsspannung (Schwellspannung der Dioden 5 und 6) und ausreichend großem Ladewiderstand — als hinreichend linear anzusehen.

**Patentansprüche**

1. Schaltung zur Kontrolle des Batteriezustands bei einem Herzschrittmacher mit einem Spannungsdetektor (8), der ein Signal abgibt, sobald eine den Batteriezustand charakterisierende Größe einen Grenzwert passiert, gekennzeichnet durch

Mittel (13) zum wiederholten Trennen der Batterie (4) von einem Ladungsspeicher (15), der durch die Batterie (4) geladen wird und die Schaltungsteile des Herzschrittmachers mit Strom versorgt, für jeweils eine vorgegebene Zeitdauer, einen festen Lastwiderstand (9), dessen Wert groß gegenüber dem Innenwiderstand ($R_i$) der Batterie (4) ist und der dem Spannungsdetektor (8) eine dem den Lastwiderstand (9) durchfließenden Strom entsprechende Spannung anlegt,

Mittel (11) zum Verbinden der Batterie (4) mit dem Lastwiderstand (9) innerhalb der Zeitdauer, wobei der Strom durch den Lastwiderstand mindestens mittelbar die den Batteriezustand charakterisierende Größe bildet, und

Mittel (16) zum Speichern des von dem Spannungsdetektor (8) abgegebenen Signals.

2. Schaltung nach Anspruch 1, dadurch gekennzeichnet, daß der Lastwiderstand (9) für einen vorgegebenen Batterietyp derart bemessen ist, daß die den Batteriezustand charakterisierende Größe ($U_l$) im wesentlichen unabhängig davon ist, ob das Passieren des Grenzwertes allein durch Unterschreiten der minimal zulässigen Leerlaufspannung ($U_{EMK}$) bei zu einer frischen Batterie gehörigem Innenwiderstand ($R_i$) oder allein durch Überschreiten des maximal zulässigen Innenwiderstandes bei der ursprünglichen Leerlaufspannung der Batterie erfolgt.

3. Schaltung nach einem der vorangehenden Ansprüche, dadurch gekennzeichnet, daß Schaltmittel in der Weise vorgesehen sind, daß der Strom durch den Lastwiderstand (9) für einen Zeitraum (T) innerhalb oder während der Zeitdauer einen Kondensator (10) auflädt, der mit dem Spannungsdetektor (8) elektrisch verbunden ist.

4. Schaltung nach Anspruch 3, dadurch gekennzeichnet, daß als Mittel zum Speichern ein Flip-Flop (16) vorgesehen ist, das zu Beginn der genannten Zeitdauer gesetzt und jeweils zurückgesetzt wird, wenn die Spannung am Kondensator (10) innerhalb des Zeitraums den vorgegebenen Grenzwert überschreitet.

5. Schaltung nach einem der Ansprüche 3 oder 4, dadurch gekennzeichnet, daß die vorgegebene Zeitdauer und der Zeitraum (T) zeitlich zusammenfallen.

6. Schaltung nach einem der vorangehenden Ansprüche, dadurch gekennzeichnet, daß die vorgegebene Zeitdauer oder der Zeitraum jeweils von einem durch den Herzschrittmacher ausgelösten Stimulationsimpuls abgeleitet wird.

7. Schaltung nach einem der Ansprüche 1 bis 5, dadurch gekennzeichnet, daß die vorgegebene Zeitdauer oder der Zeitraum jeweils durch mindestens einen CLOCK-Impuls des digitalen Zeitgebers zum Auslösen von Stimulationsimpulsen festgelegt wird.

**Claims**

1. A circuit for monitoring the battery state for a heart pacemaker, with a voltage detector (8) which produces an output signal if a value representing the battery state passes a limit value, characterised by means (13) for repeated separation for a predetermined period of the battery (4) from a charging store (15), which is charged by the battery (4) and which supplies the circuit parts of the pacemaker, a fixed load resistor (9) which has a high resistance relative to the internal resistance ($R_i$) of the battery (4) and which applies to the voltage detector (8) a voltage corresponding to the current flowing in the load resistor (9) means (11) for connecting the battery (4) with the load resistor (9) within the predetermined period, so that the current through the load impedance forms at least indirectly the value representing the battery state, and means (16) for storing the output signal from the voltage detector (8).

2. A circuit according to claim 1, characterised in that the load resistor (9) has a magnitude set for a given battery type so that the value ($U_l$) representing the battery state is substantially independent of whether the passing of the limit value occurs solely due to a shortfall of the permissible no-load voltage ($U_{EMK}$) for an internal resistance ($R_i$) of a fresh battery or solely due to an overshoot of the maximum permissible internal resistance for the original no-load voltage of the battery.

3. A circuit according to any of the preceding claims, characterised in that the switching means is arranged such that the current through the load resistor (9) charges a capacitor (10) for a time period (T) within or during said predetermined period, the capacitor being electrically connected to the voltage detector (8).

4. A circuit according to claim 3, characterised in that the storage means is a bistable (16) which is set at the beginning of said predetermined period and is re-set if the voltage on the capacitor (10) falls below the pre-determined limit value within the period.

5. A circuit according to one of claims 3 or 4, characterised in that the predetermined period and the time period (T) are the same period.

6. A circuit according to one of the previous claims, characterised in that the predetermined period or the time period are derived from a stimulation pulse produced in the pacemaker.

7. A circuit according to one of claims 1 to 5, characterised in that the predetermined period or the time period are determined by at least one clock pulse from the digital clock for controlling the stimulation pulses.

## Revendications

1. Circuit pour le contrôle de l'état de la batterie d'un stimulateur cardiaque, comportant un détecteur de tension (8) qui délivre un signal dès qu'une grandeur caractérisant l'état de la batterie dépasse une valeur limite, ledit circuit étant caractérisé par

des moyens (13) pour la séparation séparée entre la batterie (4) et un accumulateur de charge (15), qui est chargé par la batterie (4) et délivre un courant aux éléments du circuit du stimulateur cardiaque, pendant un temps prédéterminé à chaque fois; une résistance de charge fixe (9) dont la valeur est grande par rapport à la résistance interne ($R_i$) de la batterie (4); et qui applique au détecteur de tension (8) une tension correspondant au courant circulant dans la résistance de charge (9);

des moyens (11) connectant la batterie (4) à la résistance de charge (9) pendant la durée prédéterminée, le courant circulant dans la résistance de charge constituant au moins indirectement la grandeur caractéristique de l'état de la batterie; et

des moyens (16) accumulant le signal délivré par le détecteur de tension (8).

2. Circuit selon revendication 1, caractérisé en ce que la résistance de charge (9) est dimensionnée pour un type de batterie donné, de façon que la grandeur ($U_l$) caractérisant l'état de la batterie soit pratiquement indépendante du fait que le dépassement de la valeur limite résulte de la seule chute de la tension à vide maximale admissible ($U_{fém}$) pour la résistance interne ($R_i$) d'une batterie venant d'être chargée, ou du seul dépassement de la résistance interne maximale admissible pour la tension à vide initiale de la batterie.

3. Circuit selon une des revendications 1 et 2, caractérisé en ce que des moyens de commutation sont prévus de façon que le courant circulant dans la résistance de charge (9) charge, pendant un intervalle de temps (T) ou pendant l'intervalle prédéterminé, un condensateur (10) relié électriquement au détecteur de tension (8).

4. Circuit selon revendication 3, caractérisé en ce que les moyens d'accumulation prévus sont une bascule (16), qui est remise à 1 au début de la durée précitée et remise à 0 chaque fois que la tension aux bornes du condensateur (10) dépasse le seuil prédéterminé pendant la durée prédéterminée.

5. Circuit selon une des revendications 3 ou 4, caractérisé en ce que la durée prédéterminée et l'intervalle de temps (T) coincident.

6. Circuit selon une quelconque des revendications 1 à 5, caractérisé en ce que la durée prédéterminée ou l'intervalle de temps est dérivé d'une impulsion de stimulation déclenchée par le stimulateur cardiaque.

7. Circuit selon une quelconque des revendications 1 à 5, caractérisé en ce que la durée prédéterminée ou l'intervalle de temps est fixé par au moins une impulsion de l'horloge numérique déclenchant des impulsions de stimulation.

Fig. 1

Fig. 2